# EUROPÄISCHE PATENTSCHRIFT

(11) **EP 0 973 485 B1**
(45) Veröffentlichungstag und Bekanntmachung des Hinweises auf die Patenterteilung: **02.05.2003**
(21) Anmeldenummer: 98931934.8
(22) Anmeldetag: 09.04.1998
(51) Int. Cl.: A61K 7/00

(54) **KOSMETISCHES UND DERMATOLOGISCHES MITTEL AUF BASIS VON HARTMAGNETISCHEN TEILCHEN**
COSMETIC AND DERMATOLOGICAL AGENT WITH A HARD-MAGNETIC PARTICLE BASE
AGENT COSMETQUE ET DERMATOLOGIQUE A BASE DE PARTICULES MAGNETIQUES DURES

(30) Priorität: 10.04.1997 DE 19715478
(43) Veröffentlichungstag der Anmeldung: 26.01.2000
(73) Patentinhaber: LANCASTER GROUP GmbH, 55116 Mainz (DE)
(72) Erfinder: ZASTROW, Leonhard, MC-98000 Monaco (MC); GOLF-BERNER, Karin, MC-98000 Monaco (MC); DOUCET, Olivier, F-06230 Villefranche s/Mer (FR)
(74) Vertreter: Walter, Wolf-Jürgen
(86) Internationale Anmeldenummer: DE9801074
(87) Internationale Veröffentlichungsnummer: WO98044895

(56) Entgegenhaltungen:
- WO-A-94/00098
- WO-A-94/00109
- DE-A- 4 127 442
- DE-A- 4 325 071
- PATENT ABSTRACTS OF JAPAN vol. 14, no. 456 (C-0765), 2. Oktober 1990 & JP 02 180838 A (NITTA GELATIN INC), 13. Juli 1990

## Beschreibung

Die Erfindung betrifft ein neues kosmetisches und dermatologisches Mittel, das sich insbesondere zur Behandlung von empfindlichem Gewebe und Fettgewebe, zur Wundbehandlung und zur Haarbehandlung eignet.

Aus der DE-C-4325071 ist ein Präparat zur Durchblutungsförderung bekannt, das hartmagnetische Einbereichsteilchen mit einer Koerzitiv-Feldstärke von 2,387•10⁵ A/m - 3,979•10⁵ A/m (3000 bis 5000 Oerstedt) und mit Korngrößen im Bereich von 600 bis 1200 nm neben kosmetischen oder pharmazeutischen Trägerstoffen enthält. Darin genannt sind beispielsweise Barium- und Strontiumhexaferrite als Einbereichsteilchen, die insgesamt eine durchblutungsfördernde Wirkung ausüben. Die Einbereichsteilchen können auch in asymmetrischen lamellaren Aggregaten aus Fluorcarbonen und Phospholipiden verkaspelt vorliegen, um besser in die tieferen Schichten der Haut eindringen zu können.

Der Erfindung liegt die Aufgabe zugrunde, bestimmte Eigenschaften der bekannten Präparate wesentlich zu verbessern.

Es wurde nun gefunden, daß eine überraschend hohe Wundheilungswirkung und entzündungswidrige Wirkung erreicht werden kann und auch die Haarwuchs-stimulierende Wirkung derartiger Präparate in einem unerwarteten Maße erhöht werden kann, wenn die Teilchengröße der hartmagnetischen Einbereichsteilchen im Bereich von 100 bis 550 nm liegt und wenn zusätzlich neben den asymmetrischen lamellaren Aggregaten, die mit Einbereichsteilchen bzw. Magnetteilchen beladen sind, solche asymmetrischen lamellaren Aggregate vorhanden sind, die vollständig mit Sauerstoff beladen sind, und der Anteil der letzteren wenigstens 2,5 Gew-% beträgt, vorzugsweise wenigstens 10 Gew-%.

Die Erfindung betrifft daher eine kosmetisches und dermatologisches Mittel auf Basis von hartmagnetischen Teilchen und von asymmetrischen lamellaren Aggregaten, die aus Phospholipiden und Fluorcarbonen bestehen, das dadurch gekennzeichnet ist, daß es enthält
(a1) hartmagnetische Teilchen, die aus der Gruppe ausgewählt sind, die aus Bariumhexaferrit-Einkristallen, Strontiumhexaferrit-Einkristallen, Samarium-Cobalt-Teilchen (SmCo) und Neodym-Eisen-Bor-Teilchen (Nd₂Fe₁₄B) mit jeweils einer Teilchengröße im Bereich von 80 bis 550 nm besteht, und einer Koerzitiv-Feldstärke der Teilchen im Bereich von 7,957•10⁴ A/m - 1,591•10⁶ A/m (1000 bis 20000 Oerstedt);
   oder
(a2) hartmagnetische Teilchen gemäß (a1), verkapselt in wäßrigen Liposomen oder asymmetrischen lamellaren Aggregaten oder einem Gemisch von beiden, wobei die asymmetrischen lamellaren Aggregate aus natürlichen Phospholipiden mit einem Phosphatidylcholingehalt von 30 bis 99 Gew-% und Fluorcarbonen bestehen; oder ein Gemisch von (a1) und (a2); und
(b) getrennt neben (a1) oder (a2) oder deren Gemischen zusätzlich asymmetrische lamellare Aggregate, bestehend aus natürlichen Phospholipiden mit einem Phosphatidylcholingehalt von 30 bis 99 Gew-% und Fluorcarbonen, wobei die asymmetrischen lamellaren Aggregate mit Sauerstoff bis zur Sättigungsgrenze beladen sind;
(c) kosmetische oder dermatologische Trägerstoffe; und wahlweise
(d) kosmetische oder pharmakologische Wirkstoffe.

Dabei liegt der Anteil der Teilchen gemäß
(a1) oder (a2) oder deren Gemischen im Bereich von 0,001 bis 50 Gew-%,
(b) im Bereich von 2,5 bis 70 Gew.%,
(c) im Bereich von 5,0 bis 80 Gew-% und gegebenenfalls
(d) im Bereich von 0,5 bis 75 Gew-%, bezogen jeweils auf die Gesamtmasse des Mittels.

Bevorzugt sind hartmagnetische Teilchen aus Bariumhexaferrit.

Vorzugsweise liegt der Anteil pharmakologischer Wirkstoffe im Bereich von 0,1 bis 10 Gew-%, und der Anteil kosmetischer Wirkstoffe im Bereich von 0,5 bis 75 Gew-%.

Daß eine Verringerung der Teilchengröße auf 100 bis 550 nm bei den Einbereichsteilchen möglich ist, war für den Fachmann deshalb überraschend, weil mit sinkender Teilchengröße auch die Gefahr der Agglomerierung derartiger Magnetteilchen wächst. Außerdem war mit einer schwächeren Magnetfeldwirkung und damit mit einer verringerten Wirksamkeit zu rechnen. Vermutlich infolge des Charakters der Magnetteilchen als Einkristalle (Einbereichsteilchen) tritt eine solche Agglomerierung jedoch beim Vermischen mit den asymmetrischen lamellaren Aggregaten nicht oder nur unwesentlich auf oder wirkt sich nicht wesentlich aus. Die Magnetfeldwirkung ist tatsächlich schwächer, dennoch ist unerwartet eine bessere Wirksamkeit vorhanden. Ohne an eine Theorie gebunden sein zu wollen, gibt es möglicherweise Wechselwirkungen mit den zusätzlich vorhandenen asymmetrischen lamellaren Aggregaten, die allein und vollständig mit Sauerstoff beladen sind. Deren Anteil muß jedoch wenigstens 2,5 Gew-%, bezogen auf die Gesamtzusammensetzung, betragen, wenn die vermutete Wechselwirkung auftreten soll.

Die Beladung der asymmetrischen lamellaren Aggregate mit Sauerstoff erfolgt je nach ausgewähltem Fluorcarbon oder Fluorcarbongemisch vorteilhaft bis zur Sättigungsgrenze, kann jedoch auch darunter liegen. Ein bevorzugter Partialdruck liegt beispielweise im Bereich von 10 bis 40 mPa (80 bis 300 mmHg).

Vorteilhaft liegt der Gehalt an den mit Sauerstoff beladenen Aggregaten im Bereich von 10 bis 50 Gew-%.

Ein bevorzugtes erfindungsgemäßes Mittel ist ein solches, das aus
(a) Bariumhexaferrit-Einkristallen mit einer Teilchengröße im Bereich von 100 bis 500 nm und einer Koerzitiv-Feldstärke der Teilchen im Bereich von 7,957•10⁴ A/m - 3,183•10⁴ A/m (1000 bis 4000 Oe). verkapselt in wäßrigen asymmetrischen lamellaren Aggregaten, in einem Anteil von 0,01 bis 10 Gew-% und
(b) zusätzlich asymmetrischen lamellaren Aggregaten, die allein mit Sauerstoff bis zum Sättigungspartialdruck, vorzugsweise 10 bis 40 mPa (80 bis 300 mmHg) beladen sind und
(c) kosmetischen oder dermatologischen Trägerstoffen besteht.

Ein solches bevorzugtes Mittel enthält einen Anteil der Teilchen von (a) von 0,1 bis 5 %, (b) von 5 bis 30 % und (c) von 0,1 bis 5 Gew-%.

Die erfindungsgemäße Teilchengröße beträgt 80 - 550 nm für die hartmagnetischen Teilchen. Es können auch geringe Mengen größerer oder kleinerer Teilchen enthalten sein (unter 5 %), wesentlich ist jedoch, daß die mittlere Teilchengröße D₅₀ etwa 250 nm beträgt. Das bedeutet, daß wenigstens 50 % der in einer Emulsion enthaltenen hartmagnetischen Teilchen in der Größenordnung 250 µm liegen. Eine typische Teilchengrößenverteilung nach der Erfindung ist beispielsweise
15 % 80 bis 100 nm
55 % 100 bis 250 nm
30 % 250 bis 350 nm.

Unter dem hier verwendeten Begriff "Fluorcarbone" werden perfluorierte oder hochfluorierte Kohlenstoffverbindungen oder Gemische verstanden, die in der Lage sind, Gase wie Sauerstoff und Kohlendioxid zu transportieren. Hochfluorierte Kohlenwasserstoffverbindungen sind im Sinne dieser Erfindung solche, bei denen die meisten Wasserstoffatome durch Fluoratome ersetzt sind, so daß bei weiterem Ersatz nicht notwendigerweise die Fähigkeit zum Gastransport erhöht wird. Dies wird meist dann erreicht, wenn etwa bis zu 90 % der Wasserstoffatome durch Fluoratome ersetzt sind. Bevorzugt im Sinne der vorliegenden Erfindung sind Fluorcarbone, bei denen wenigstens 95 % der Wasserstoffatome ersetzt sind, bevorzugter 98 % und am bevorzugtesten 100 %.

Es können eine Vielzahl von Fluorcarbonen eingesetzt werden, z.B. aliphatische geradkettige und verzweigte Fluoralkane, monooder bicyclische und gegebenenfalls fluoralkylsubstituierte Fluorcycloalkane, perfluorierte aliphatische oder dicyclische Amine, Bis-(perfluoralkyl)-Ethene, Perfluorpolyether oder deren Gemische. Besonders bevorzugt sind solche Fluorcarbone wie Perfluordecalin, F-Butyltetrahydrofuran, Perfluortributylamin, Perfluoroctylbromid, Bis-Fluor(butyl)-ethen oder Bis-Fluor(hexyl)ethen oder C₆-C₉-Perfluoralkane.

Den erfindungsgemäßen Zusammensetzungen können zusätzlich weitere kosmetische oder pharmakologisch wirksame Substanzen zugesetzt werden. Als kosmetisch wirksame Substanzen sind besonders geeignet Vitamine, Enzyme, β-1,3-Glucan, β-1,6-Glucan, Carboxymethyl-Glucan (z.B. CM-Glucan®), Planzenextrakte usw.

Als pharmakologisch besonders wirksame Substanzen sind besonders geeignet Heparin, Acetylsalicylsäure, Piroxicam, Miroxicam, östrogene.

Die kosmetischen oder pharmakologisch wirksamen Substanzen können in der wäßrigen Phase der Emulsion vorhanden sein; sie können auch gesondert in Liposomen oder asymmetrischen lamellaren Aggregaten wie die hartmagnetischen Teilchen gemäß (a2) enthalten sein. Beispielsweise ist Acetylsalicylsäure in der wäßrigen Emulsionsphase enthalten und erhöht dadurch zusätzlich die Stabilität der Emulsion.

Als kosmetische oder dermatologische Träger- und Wirkstoffe sind geeignet Wasser, Öle, Emulgatoren, Gele, Liposome sowie spezielle Komponenten, wie Phospholipide, Carbomer, Cetearylakohol, Cetylalkohol, Isopropylmyristat, Iospropylpalmitat, Isopropylstearat, Octylstearat usw.

Als weiteren Wirkstoff kann das Präparat vorteilhaft Kaolin gemäß WO96/17588 enthalten, der mit sphärischen TiO₂- oder SiO₂-Teilchen mit einer Teilchengröße <5 µm modifiziert ist, wobei die sphärischen Teilchen einen Anteil an der Kaolinmischung von 0,5 bis 10 Gew-% haben. Das Präparat erhält dadurch ein sehr weiches Hautgefühl und eine zusätzliche entzündungswidrige Wirksamkeit.

Der modifizierte Kaolin kann einen Anteil von 0,1 bis 6 Gew-% haben, bezogen auf die Gesamtmenge des Präparates.

Die Herstellung der erfindungsgemäßen Mittel erfolgt in der Weise, daß zuerst die hartmagnetischen Einbereichsteilchen gemäß (a1) mit asymmetrischen lamellaren Aggregaten bei ca. 10.000 U/Min und 30-35 °C vermischt werden und anschließend die Beladung des Gemisches mit vorzugsweise reinem Sauerstoff bis zum gewünschten 02-Partialdruck erfolgt. Danach wird die Vermischung mit den kosmetischen oder dermatologischen Trägerstoffen, die gegebenenfalls bereits andere Wirkstoffe enthalten können, durchgeführt.

Eine weitere Ausführungsform besteht darin, daß zuerst mit hartmagnetischen Einbereichsteilchen beladene asymmetrische lamellare Aggregate hergestellt werden, in denen die Magnetpartikel mit den Phospholipiden und den Fluorcarbonen sowie weiteren Hilfsstoffen vermischt werden. Danach erfolgt die Herstellung von asymmetrischen lamellaren Aggregaten ohne Magnetpartikel und die Beladung dieser Aggregate mit Sauerstoff, indem man reinen Sauerstoff z. B. bis zur Sättigung der Emulsion durch diese hindurch perlen läßt. Im Anschluß daran werden die beiden Aggregattypen miteinander vermischt.

Für den Fall, daß weitere kosmetische oder dermatologische Wirkstoffe im Endprodukt enthalten sein sollen, können diese zusammen mit den Magnetpartikeln in die asymmetrischen lamellaren Aggregate aufgenommen werden, d.h. in dem Perfluorcarbon, das das Innere der Aggregate bildet. Dies ist vorteilhaft bei solchen Stoffen, die in der wäßrigen Phase kristallisieren würden. Ist das nicht der Fall, wie z.B. bei Acetylsalicylsäure, können sie auch in der wäßrigen Phase der Emulsion aufgenommen werden, wie oben bereits erläutert.

Die asymmetrischen lamellaren Aggregate selbst werden wie folgt hergestellt:

Natürliche Phospholipide mit einem Gehalt an Phosphatidylcholin von 30 bis 99 Gew.-% werden mit einem Fluorcarbon, z. B. Perfluordecalin bei ca. 10000 U/Min homogenisiert. Anschließend werden Hilfs- und Konservierungsstoffe sowie Wasser zugegeben, und es wird nochmals unter Kühlung gerührt und bei ca. 20000 U/Min homogenisiert.

Unter dem Begriff "Einbereichsteilchen" werden Einkristalle mit von Hause aus einheitlicher magnetischer Orientierung verstanden. Besonders bevorzugt in der vorliegenden Erfindung als hartmagnetische Einbereichsteilchen sind Barium- oder Strontiumhexaferrite, die vorteilhafterweise nicht dotiert sind. Die Herstellung dieser undotierten Barium- oder Strontiumhexaferrite erfolgt nach bekannten Verfahren, z.B. nach der Glaskristallisationstechnik durch Züchtung von Einkristallen aus einer abgeschreckten Glasschmelze. Ein geeignetes Glas dafür befindet sich im Dreistoffsystem BaO-Fe₂O₃-B₂O₃. Es setzt sich vorteilhaft zusammen aus 20 bis 50 Gew.-% Fe₂O₃, 30 bis 50 Gew.-% BaO und 20 bis 50 Gew.-% B₂O₃.

Das Durchmesser/Dickenverhältnis der Kristalle von Bariumhexaferrit oder strontiumhexaferrit liegt im allgemeinen bei 3 : 1 bis 10 : 1.

Eine weitere Wirkung der erfindungsgemäßen Zusammensetzung besteht darin, daß das Haarwachstum im Verhältnis zu einer Präparation gemäß Beispiel 18 der DE-C-4325071 wesentlich höher liegt. Unerwartet wurde festgestellt, daß die Zahl der Haare in der Anagenphase, also in Wachstumsphase der Haare, um signifikante Werte von bis zu etwa 20 % höher liegt als normal zu erwarten ist. Je nach Anteil der hartmagnetischen Einbereichsteilchen und der Sauerstoff-beladenen asymmetrischen lamellaren Aggregate und der Behandlungsdauer kann bei einer normalen Wachstumsgeschwindigkeit zu dickerem Haar und zu einem erhöhten Anteil von Haar in der Anagenphase zuungunsten der Katagen-Phase und der Teleogen-Phase führen.

Hinsichtlich der Wundheilungs- und entzündungswidrigen Wirkung wurde festgestellt, daß gegenüber den reinen Aggregaten mit hartmagnetischen Teilchen bei gleicher Konzentration der hartmagnetischen Teilchen ein Gemisch: Aggregate mit hartmagnetischen Teilchen + Aggregate mit Sauerstoffsättigung eine wesentlich verbesserte entzündungswidrige Wirkung zeigen. Diese Wirkung kann durch Aufnahme bestimmter pharmakologischer Wirkstoffe noch verbessert werden. Dabei wurde festgestellt, daß neben einer durch die hartmagnetischen Teilchen bewirkten Verbesserung der Mikrozirkulation auch eine verbesserte Aufnahme der Wirkstoffe durch das Gewebe erfolgt gegenüber der sonst bei dieser Applikationsform zu erwartenden Wirkung, und somit eine insgesamt potenzierte Wirksamkeit (synergismus) zu beobachten ist.

Das Einbringen des sauerstoffbeladenen Gemisches in die Trägerstoffe zur Herstellung einer Dispersion erfolgt vorteilhaft unter mäßigem Rühren (300 bis 3000 U/Min) und bei Temperaturen unter 50 °C, vorzugsweise zwischen 10 und 40 °C, um einen hohen Sauerstoffgehalt beizubehalten, vorzugsweise einen Sauerstoffgehalt in Nähe des Sättigungspartialdruckes.

Die Erfindung soll nachstehend durch Beispiele näher erläutert werden.

### Beispiel 1

1A) Herstellung der Aggregate mit hartmagnetischen Teilchen
   Die hartmagnetischen Teilchen, bestehend aus Bariumhexaferrit, mit einer durchschnittlichen Teilchengröße D₅₀ von 100 bis 250 nm wurden in Wasser homogenisiert, Carbomer wurde zugegeben und es wurde neutralisiert. Anschließend erfolgte die Zugabe eines Fluorcarbons, eines Phospholipids mit einem Gehalt an Phosphatidylcholin etwa 90 %, und es wurde homogenisiert. Danach erfolgte die Zugabe von Glycerin, Propylenglycol und des Konservierungsmittels ebenfalls unter Rühren. Insgesamt soll die Temperatur nicht über 35 °C liegen.
1B) Herstellung der O₂-gesättigten Aggregate
   Die Herstellung der Aggregate erfolgt durch Vermischen von Phospholipiden mit einem Gehalt an Phosphatidylcholin von 30 bis 99 Gew.-% mit einem oder mehreren Fluorcarbonen, z. B. mit Perfluordecalin. Nach weiterer Zugabe von Hilfsstoffen, wie Glycerin, Propylenglycol sowie Konservierungsmitteln und Wasser wird durch die homogenisierten, fertigen, asymmetrischen lamellaren Aggregate ein reiner Sauerstoffstrom bis zur Sättigungsgrenze geleitet.
   Die magnetischen Einbereichsteilchen enthaltenden Aggregate können mit den sauerstoffgesättigten Aggregaten frei vermischt werden.
1C) Herstellung einer Emulsion mit hartmagnetischen Partikeln Hartmagnetische Teilchen werden in Wasser homogenisiert, und danach wird eine Emulsionsgrundlage zugegeben. Anschließend erfolgt das Vermischen der mit den Magnetpartikeln versetzten Emulsionen mit den gemäß Beispiel 1B) hergestellten sauerstoffgesättigten asymmetrischen lamellaren Aggregate.
1D) Herstellung von Mitteln mit kosmetischen oder pharmakologischen Wirkstoffen

Der Wirkstoff wird in Wasser gelöst oder suspendiert, Carbomer wird hinzugesetzt, und es erfolgt eine Neutralisierung. Im Anschluß daran werden die hartmagnetischen Teilchen zugegeben und das ganze homogenisiert. Im Anschluß daran wird ein Fluorcarbon oder Fluorcarbongemisch sowie ein natürliches Phospholipid mit hohem Phosphatidylcholingehalt langsam zugegeben und homogenisiert. Nach weiterer Zugabe von Hilfs- und Konservierungsstoffen erfolgt ein Rühren bis zur Homogenität.

### Beispiel 2 Brustcreme für übersensible Haut

| Phase A | |
|---|---|
| Wasser | q.s. |
| Propylenglycol | 0,5 % |
| Glycerin | 0,5 % |
| Acrylate/C 10-30 Alkylacrylate Crosspolymer | 0,3 % |

| Phase B | |
|---|---|
| Cetearyl Alkohol | 2,5 % |
| Cetearyl Alkohol & Cetyl Palmitate | 1,5 % |
| Octyl Stearate | 1,5 % |

| Phase C | |
|---|---|
| Triethanolamin | 0,3 % |
| Konservierungsmittel | 0,4 % |

| Phase D | |
|---|---|
| Babassu Oil | 1 % |
| Fragrance | 0,2 % |
| Aggregate gemäß Beispiel 1A | 20 % |
| Aggregate gemäß Beispiel 1B | 10 %. |

Die Phasen A und B wurden jeweils getrennt auf 60 bis 70 °C erhitzt, die Phase C wurde gemischt und neutralisiert. Die Phasen A, B und C wurden unter Rühren der Phase D zugesetzt.

### Beispiel 3 Haarmaske

| Phase A | |
|---|---|
| Wasser | q.s. |
| Glycerin | 1 % |
| Acrylates/C10-30 Alkyl Acrylate Crosspolymer | 1 % |

| Phase B | |
|---|---|
| Triethanolamin | 1 % |

| Phase C | |
|---|---|
| Konservierungsmittel | 0,3 % |
| Aggregate gemäß Beispiel 1A | 10 % |
| Aggregate gemäß Beispiel 1B | 10 % |
| Melanin löslich | 0,1 % |
| Hefeextrakt | 1 % |
| Fragrance | 0,5 % |

Die Phase A wurde homogenisiert, die Phase B neutralisiert, und beide wurden der Phase C unter Rühren bei einer Temperatur unter 40 °C zugesetzt.

### Beispiel 4 Heparinsalbe

| | |
|---|---|
| Wasser | q.s. |
| Heparin | 1 % |
| Carbomer | 2 % |
| Natriumhydroxid | 2 % |
| Phospholipid | 9 % |
| Perfluordecalin | 20 % |
| Aggregate gemäß Beispiel 1A | 2 % |
| Aggregate gemäß Beispiel 1B | 5 % |
| Glycerin | 1 % |
| Konservierungsmittel | 0,1 %. |

In das Perfluordecalin wurden unter starkem Rühren Heparin und die Aggregate gemäß Beispiel 1A zugegeben, wobei die Temperatur bei oder unter 35 °C gehalten wurde. Danach erfolgte in üblicher Weise die Zugabe der anderen Rohstoffe. Zum Schluß erfolgte der Zusatz der Aggregate gemäß Beispiel 1B.

### Beispiel 5 ASS-Salbe

Es wurden 1 % Acetylsalicylsäure unter gutem Rühren in Wasser gelöst. Danach wurde das Fluorcarbon mit einem Phospholipid mit einem Gehalt an Phosphatidylcholin von 40 Gew-% verrührt, Glycerin hinzugegeben und mit Wasser homogenisiert. Das ASS-Homogenisat wurde in das Homogenisat mit dem Fluorcarbon gegeben und für etwa 20 Minuten bei weniger als 35 °C homogenisiert. Der Anteil des Fluorcarbons betrug 40 %, des Phospholipids 20 %. Anschließend wurde das Gemisch mit den gemäß Beispiel 1C hergestellten asymmetrischen lamellaren Aggregate vermischt, die einen Anteil am Gesamtgemisch von 8 Gew.-% einnahmen.

## Patentansprüche

1. Kosmetisches und dermatologisches Mittel auf Basis von hart-magnetischen Teilchen und von asymmetrischen lamellaren Aggregaten, die aus Phospholipiden und Fluorcarbonen bestehen, **dadurch gekennzeichnet, daß** es enthält
(a1) hartmagnetische Teilchen, die aus der Gruppe ausgewählt sind, die aus Bariumhexaferrit-Einkristallen, Strontiumhexaferrit-Einkristallen, Samarium-Cobalt-Teilchen (SmCo) und Neodym-Eisen-Bor-Teilchen (Nd₂Fe₁₄B) mit jeweils einer Teilchengröße im Bereich von 80 bis 550 nm besteht, und einer Koerzitiv-Feldstärke der Teilchen im Bereich von 7,957•10⁴ A/m - 1,591•10⁶ A/m (1.000 bis 20.000 Oerstedt);
oder
(a2) hartmagnetische Teilchen gemäß (a1), verkapselt in wäßrigen Liposomen oder asymmetrischen lamellaren Aggregaten oder einem Gemisch von beiden, wobei die asymmetrischen lamellaren Aggregate aus natürlichen Phospholipiden mit einem Phosphatidylcholingehalt von 30 bis 99 Gew-% und Fluorcarbonen bestehen;
oder ein Gemisch von (a1) und (a2); und
(b) getrennt neben (a1) oder (a2) oder deren Gemischen zusätzlich asymmetrische lamellare Aggregate, bestehend aus natürlichen Phospholipiden mit einem Phosphatidylcholingehalt von 30 bis 99 Gew-% und Fluorcarbonen, wobei die asymmetrischen lamellaren Aggregate mit Sauerstoff bis zum Sättigungspartialdruck beladen sind; und
(c) kosmetische oder dermatologische Trägerstoffe; und wahlweise
(d) kosmetische oder pharmakologische Wirkstoffe;
und wobei der Anteil der Teilchen gemäß
(a1) oder (a2) im Bereich von 0,001 bis 50 Gew-%,
(b) im Bereich von 2,5 bis 70 Gew.%,
(c) im Bereich von 5 bis 80 Gew-% und gegebenenfalls
(d) im Bereich von 0,5 bis 75 Gew-% liegt, bezogen jeweils auf die Gesamtmasse des Mittels.

2. Mittel nach Anspruch 1, **dadurch gekennzeichnet, daß** es aus
(a) Bariumhexaferrit-Einkristallen mit einer Teilchengröße im Bereich von 80 bis 550 nm und einer Koerzitiv-Feldstärke der Teilchen im Bereich von 7,957•10⁴ A/m - 3,979•10⁵ A/m (1000 bis 5000 Oe), verkapselt in wäßrigen asymmetrischen lamellaren Aggregaten, in einem Anteil von 0,01 bis 10 Gew-% und
(b) zusätzlich asymmetrischen lamellaren Aggregaten, die allein mit Sauerstoff bis zum Sättigungspartialdruck beladen sind und
(c) kosmetischen oder dermatologischen Trägerstoffen besteht.

3. Mittel nach Anspruch 2, **dadurch gekennzeichnet, daß** es einen pharmakologischen Wirkstoff enthält, ausgewählt aus der Gruppe, die aus Heparin, Acetylsalicylsäure, Piroxicam, Miroxicam und östrogenen besteht.

4. Mittel nach Anspruch 2, **dadurch gekennzeichnet, daß** es einen kosmetischen Wirkstoff enthält, ausgewählt aus der Gruppe, die aus Vitaminen, Enzymen, Vitamin-Enzymgemischen der Ultraschallbehandlung von Hefen, β-1,3-Glucan und Carboxymethyl-Glucan besteht.

5. Mittel nach einem der Ansprüche 1 bis 4, **dadurch gekennzeichnet, daß** der Anteil der mit Sauerstoff im wesentlichen vollständig beladenen asymmetrischen lamellaren Aggregate gemäß Anspruch 1(b) im Bereich von 10 bis 40 Gew-% liegt.

6. Mittel nach einem der Ansprüche 1 bis 5, **dadurch gekennzeichnet, daß** der Anteil der hartmagnetischen Einbereichsteilchen im Bereich von 0,1 bis 30 Gew-%, insbesondere im Bereich von 0,5 bis 10 Gew-% liegt.

7. Mittel nach einem der Ansprüche 1 bis 6, **dadurch gekennzeichnet, daß** der Sauerstoffpartialdruck in den beladenen asymmetrischen lamellaren Aggregaten nach der Beladung 10 bis 40 mPa (180 bis 300 mmHg) beträgt.

8. Verfahren zur Herstellung eines kosmetischen oder dermatologischen Mittels auf Basis von hart-magnetischen Teilchen und von asymmetrischen lamellaren Aggregaten, die aus Phospholipiden und Fluorcarbonen bestehen, **dadurch gekennzeichnet, daß** man
(a1) hartmagnetische Teilchen herstellt, die aus der Gruppe ausgewählt sind, die aus Bariumhexaferrit-Einkristallen, Strontium-hexaferrit-Einkristallen, Samarium-Cobalt-Teilchen (SmCo) und Neodym-Eisen-Bor-Teilchen (Nd₂Fe₁₄B) besteht, mit jeweils einer Teilchengröße im Bereich von 80 bis 550 nm,
und einer Koerzitiv-Feldstärke der Teilchen im Bereich von 7,957•10⁴ A/m - 1,591•10⁶ A/m (1.000 bis 20.000 Oerstedt);
oder
(a2) hartmagnetische Teilchen gemäß (a1) herstellt und in wäßrigen Liposomen oder asymmetrischen lamellaren Aggregaten oder einem Gemisch von beiden verkapselt, wobei die asymmetrischen lamellaren Aggregate aus natürlichen Phospholipiden mit einem Phosphatidylcholingehalt von 30 bis 99 Gew-% und Fluorcarbonen bestehen;
oder ein Gemisch von (a1) und (a2) herstellt;
und
(b) getrennt neben (a1) oder (a2) oder deren Gemischen zusätzlich asymmetrische lamellare Aggregate herstellt, bestehend aus natürlichen Phospholipiden mit einem Phosphatidylcholingehalt von 30 bis 99 Gew-% und Fluorcarbonen, und
(b1) diese gemäß (b) hergestellten zusätzlichen asymmetrischen lamellaren Aggregate mit Sauerstoff bis zum Sättigungspartialdruck belädt und anschließend mit den Aggregaten (a2) oder dem Gemisch von (a2) mit (a1) vermischt;
oder
(b2) die gemäß (a2) hergestellten Aggregate oder das Gemisch von (a2) mit (a1) mit den nach (b) hergestellten Aggregaten vermischt und dann beide Aggregattypen gemeinsam mit Sauerstoff bis zum Sättigungspartialdruck belädt;
und
das gemäß (b1) oder (b2) hergestellte sauerstoffbeladene Gemisch mit kosmetischen oder dermatologischen Trägerstoffen (c) zu einer Dispersion vermischt; und wahlweise kosmetische oder pharmakologische Wirkstoffe (d) in das Gemisch einbringt;
und wobei der Anteil der Teilchen gemäß
(a1) oder (a2) im Bereich von 0,001 bis 50 Gew-%,
(b) im Bereich von 2,5 bis 70 Gew.%,
(c) im Bereich von 5 bis 80 Gew-% und gegebenenfalls
(d) im Bereich von 0,5 bis 75 Gew-% liegt, bezogen jeweils auf die Gesamtmasse des Mittels.

9. Verfahren nach Anspruch 8, **dadurch gekennzeichnet, daß** die Wirkstoffe (d) zusammen mit den hartmagnetischen Teilchen in Liposome oder asymmetrische lamellare Aggregate in Stufe (a2) eingebracht werden.

10. Verfahren nach Anspruch 8 oder 9, **dadurch gekennzeichnet, daß** das Einbringen des sauerstoffbeladenen Gemisches in die Trägerstoffe zur Herstellung einer Dispersion unter mäßigem Rühren und bei Temperaturen zwischen 10 und 50 °C erfolgt zur Beibehaltung eines hohen Sauerstoffgehaltes.

## Claims

1. A cosmetic and dermatological preparation based on magnetically hard particles and asymmetrical lamellar aggregates consisting of phospholipids and fluorocarbons, comprising of
(a1) magnetically hard particles selected from the group consisting of barium hexaferrite single crystals, strontium hexaferrite single crystals, samarium-cobalt particles (SmCo) and neodymium-iron-boron particles (Nd₂Fe₁₄B), each with a particle size in the range of 80 to 550 nm, and the particles have a coercive field strength in the range of 7.957 · 10⁴ A/m-1.591 · 10⁶ A/m (1,000 to 20,000 Oerstedt);
or
(a2) magnetically hard particles according to (a1), encapsulated in aqueous liposomes or asymmetrical lamellar aggregates or a mixture of the two, where the asymmetrical lamellar aggregates consist of natural phospholipids with a phosphatidylcholine content of 30 to 99 wt% and fluorocarbons;
or a mixture of (a1) and (a2);
and
(b) separately in addition to (a1) or (a2) or mixtures thereof, it also contains asymmetrical lamellar aggregates consisting of fluorocarbons and natural phospholipids having a phosphatidylcholine content of 30 to 99 wt%, where the asymmetrical lamellar aggregates are loaded with oxygen to the saturation partial pressure; and
(c) cosmetic or dermatological excipients; and optionally
(d) cosmetic or pharmacological active ingredients; and where the amount of particles according to
(a1) or (a2) is in the range of 0.001 to 50 wt%,
(b) is in the range of 2.5 to 70 wt%,
(c) is in the range of 5 to 80 wt%, and optionally
(d) is in the range of 0.5 to 75 wt%, based on the total weight of the preparation.

2. A preparation according to Claim 1, wherein it consists of
(a) barium hexaferrite single crystals with a particle size in the range of 80 to 550 nm, the particles having a coercive field strength in the range of 7.957 · 10⁴ A/m - 3.979 · 10⁵ A/m (1,000 to 5,000 Oe), encapsulated in aqueous asymmetrical lamellar aggregates, in an amount of 0.01 to 10 wt% and
(b) additionally, asymmetrical lamellar aggregates which are loaded only with oxygen up to the saturation partial pressure, and
(c) cosmetic or dermatological excipients.

3. A preparation according to Claim 2, wherein it contains a pharmacological active ingredient selected from the group consisting of heparin, aspirin (acetylsalicylic acid), piroxicam, miroxicam and estrogens.

4. A preparation according to Claim 2, wherein contains a cosmetic active ingredient selected from the group consisting of vitamins, enzymes, vitamin-enzyme mixtures from ultrasonic treatment of yeasts, beta-1,3-glucan and carboxymethyl-glucan.

5. A preparation according to one of Claims 1 through 4, wherein the amount of asymmetrical lamellar aggregates according to Claim 1(b) loaded with oxygen essentially to saturation is in the range of 10 to 40 wt%.

6. A preparation according to one of Claims 1 through 5, wherein the amount of magnetically hard single-domain particles is in the range of 0.1 to 30 wt%, especially in the range of 0.5 to 1 wt%.

7. A preparation according to one of Claims 1 through 6, wherein the oxygen partial pressure in the loaded asymmetrical lamellar aggregates is 10 to 40 mPa (180 to 300 mm Hg) after loading with oxygen.

8. Process for the manufacture of a preparation based on magnetically hard particles and asymmetrical lamellar aggregates consisting of phospholipids and fluorocarbons, comprising incorporating
(a1) magnetically hard particles selected from the group consisting of barium hexaferrite single crystals, strontium hexaferrite single crystals, samarium-cobalt particles (SmCo) and neodymium-iron-boron particles (Nd₂Fe₁₄B), each with a particle size in the range of 80 to 550 nm, and with a coercive field strength of the particles in the range of 7.957 · 10⁴ A/m-1.591 · 10⁶ A/m (1,000 to 20,000 Oerstedt); or
(a2) magnetically hard particles according to (a1), encapsulated in aqueous liposomes or asymmetrical lamellar aggregates or a mixture of the two, where the asymmetrical lamellar aggregates consist of natural phospholipids with a phosphatidylcholine content of 30 to 99 wt% and fluorocarbons;
or a mixture of (a1) and (a2);
and separately in addition to (a1) or (a2) or mixtures thereof
(b) asymmetrical lamellar aggregates consisting of fluorocarbons and natural phospholipids having a phosphatidylcholine content of 30 to 99 wt%, where the asymmetrical lamellar aggregates are loaded with oxygen to the saturation partial pressure;
into a cosmetically or pharmacologically acceptable excipient
(c) to form a dispersion; and
with the optional addition of cosmetical or pharmacological active ingredients (d) to form said dispersion;
wherein the amount of particles according to
(a1) or (a2) is in the range of 0.001 to 50 wt%,
(b) is in the range of 2.5 to 70 wt%,
(c) is in the range of 5 to 80 wt%, and optionally
(d) is in the range of 0.5 to 75 wt%, based on the total weight of said dispersion.

9. Process according to claim 8, wherein the cosmetical or pharmacological active ingredients (d) are introduced in step (a2) together with the magnetically hard particles in liposomes or asymmetric lamellar aggregates.

10. Process according to claim 8 or 9, wherein the oxygen-charged mixtures are introduced into the excipients or carrier materials for preparing said dispersion by gentle stirring and maintaining temperatures between 10 to 50 °C for maintenance of high levels of oxygen.

## Revendications

1. Agent cosmétique et pharmaceutique à base de particules magnétiques dures et d'agrégats asymétriques lamellaires constitués de phospholipides et de fluorocarbones, **caractérisé en ce qu'**il contient
(a1) des particules magnétiques dures choisies parmi le groupe des monocristaux d'hexaferrite de baryum, monocristaux d'hexaferrite de strontium, particules de samarium - cobalt (SmCo) et particules de néodyme - fer - bore (Nd₂Fe₁₄B) avec respectivement une granulométrie allant de 80 à 550 nm,
et une intensité du champ coercitif des particules allant de 7,957.10⁴ A/m à 1,591.10⁶ A/m (1000 à 20 000 oerstedt) ;
ou
(a2) des particules magnétiques dures selon (a1), encapsulées dans des liposomes aqueux ou des agrégats asymétriques lamellaires ou un mélange des deux, les agrégats asymétriques lamellaires étant constitués de phospholipides, naturels ayant une teneur en phosphatidylcholine allant de 30 à 99 % en poids, et de fluorocarbones ;
ou un mélange de (a1) et (a2) ; et
(b) en dehors de (a1) ou (a2) ou leurs mélanges, en plus des agrégats asymétriques lamellaires constitués de phospholipides naturels ayant une teneur en phosphatidylcholine de 30 à 99 % en poids et de fluorocarbones, les agrégats asymétriques lamellaires étant chargés d'oxygène jusqu'au seuil de saturation ; et
(c) des substances support cosmétiques ou dermatologiques ; et au choix
(d) des actifs cosmétiques ou pharmacologiques ;
et la partie des particules se situant alors selon
(a1) ou (a2) ou leurs mélanges dans la plage allant de 0,001 à 50 % en poids,
(b) dans la plage allant de 2,5 à 70 % en poids,
(c) dans la plage allant de 5,0 à 80 % en poids et le cas échéant
(d) dans la plage allant de 0,5 à 75 % en poids, sur la masse totale de l'agent.

2. Agent selon la revendication 10, **caractérisé en ce qu'**il est constitué de
(a) de monocristaux d'hexaferrite de baryum ayant une granulométrie située entre 100 et 500 nm et une intensité de champ coercitif allant de 7,957.10⁴ A/m à 3,183.10⁴ A/m (1000 à 4000 oerstedt), encapsulés dans des agrégats asymétriques lamellaires aqueux, en une partie allant de 0,01 à 10 % en poids et
(b) en plus d'agrégats asymétriques lamellaires chargés uniquement d'oxygène jusqu'à pression partielle de saturation, de préférence de 10 à 40 mPa (80 à 300 mmHg) et
(c) de substances supports cosmétiques et dermatologiques.

3. Agent selon la revendication 2, **caractérisé en ce qu'**il contient un actif pharmacologique, choisi parmi le groupe constitué de l'héparine, l'acide acétylsalicylique, le piroxicam, le miroxicam et les oestrogènes.

4. Agent selon la revendication 2, **caractérisé en ce qu'**il contient un actif cosmétique choisi parmi le groupe constitué des vitamines, enzymes, mélanges vitamino-enzymatiques du traitement par ultrasons des levures, du β-1,3-glucane et du carboxyméthylglucane.

5. Agent selon l'une quelconque des revendications 1 à 4, **caractérisé en ce que** la partie des agrégats asymétriques lamellaires chargés essentiellement entièrement d'oxygène selon la revendication 1(b) se situe entre 10 et 40 % en poids.

6. Agent selon l'une quelconque des revendications 1 à 5, **caractérisé en ce que** la partie des monoparticules magnétiques dures se situe dans la plage allant de 0,1 à 30 % en poids, notamment dans la plage allant de 0,5 à 10 % en poids.

7. Agent selon l'une quelconque des revendications 1 à 6, **caractérisé en ce que** la pression partielle d'oxygène dans les agrégats asymétriques lamellaires chargés atteint après chargement 10 à 40 mPa (180 à 300 mmHg).

8. Procédé de préparation d'un agent cosmétique et pharmaceutique à base de particules magnétiques dures et d'agrégats asymétriques lamellaires constitués de phospholipides et de fluorocarbones, **caractérisé en ce que** l'on
(a1) prépare des particules magnétiques dures choisies parmi le groupe des monocristaux d'hexaferrite de baryum, monocristaux d'hexaferrite de strontium, particules de samarium - cobalt (SmCo) et particules de néodyme - fer - bore (Nd₂Fe₁₄B) avec respectivement une granulométrie allant de 80 à 550 nm,
et une intensité du champ coercitif des particules allant de 7,957.10⁴ A/m à 1,591.10⁶ A/m (1000 à 20 000 oerstedt) ;
ou
(a2) prépare des particules magnétiques dures selon (a1), encapsulées dans des liposomes aqueux ou des agrégats asymétriques lamellaires ou un mélange des deux, les agrégats asymétriques lamellaires étant constitués de phospholipides, naturels ayant une teneur en phosphatidylcholine allant de 30 à 99 % en poids, et de fluorocarbones ;
ou prépare un mélange de (a1) et (a2) ;
et
(b) prépare séparément en dehors de (a1) ou (a2) ou leurs mélanges, en plus des agrégats asymétriques lamellaires constitués de phospholipides naturels ayant une teneur en phosphatidylcholine de 30 à 99 % en poids et de fluorocarbones, et
(b1) charge les agrégats asymétriques lamellaires en sus préparés selon (b) avec de l'oxygène jusqu'à la pression partielle de saturation et les mélange ensuite avec les agrégats (a2) ou le mélange de (a2) avec (a1) ;
ou
(b2) mélange les agrégats préparés selon (a2) ou le mélange de (a2) avec (a1) avec les agrégats préparés selon (b) et ensuite charge les deux types d'agrégat ensemble avec de l'oxygène jusqu'à pression partielle de saturation ;
et
mélange le mélange chargé en oxygène préparé selon (b1) ou (b2) avec des substances support cosmétiques ou dermatologiques (c) en une dispersion ; et au choix incorpore dans le mélange des actifs cosmétiques ou pharmacologiques (d) ;
et la partie des particules se situant alors selon
(a1) ou (a2) ou leurs mélanges dans la plage allant de 0,001 à 50 % en poids,
(b) dans la plage allant de 2,5 à 70 % en poids,
(c) dans la plage allant de 5,0 à 80 % en poids et le cas échéant
(d) dans la plage allant de 0,5 à 75 % en poids, sur la masse totale de l'agent.

9. Procédé selon la revendication 8, **caractérisé en ce que** les actifs (d) sont incorporés en compagnie des particules magnétiques dures dans les liposomes ou les agrégats asymétriques lamellaires à l'étape (a2).

10. Procédé selon la revendication 8 ou 9, **caractérisé en ce que** l'incorporation du mélange chargé en oxygène dans les substances supports pour la préparation d'une dispersion s'effectue sous agitation modérée et à des températures entre 10 et 50°C pour conserver la teneur élevée en oxygène.
